(19) Europäisches Patentamt
European Patent Office
Office européen des brevets

(11) **EP 3 416 701 B1**

(12) **EUROPÄISCHE PATENTSCHRIFT**

(45) Veröffentlichungstag und Bekanntmachung des Hinweises auf die Patenterteilung:
**01.04.2020   Patentblatt 2020/14**

(21) Anmeldenummer: **17705314.7**

(22) Anmeldetag: **15.02.2017**

(51) Int Cl.:
*A61M 1/36* (2006.01)        *A61B 5/026* (2006.01)
*A61B 5/0275* (2006.01)      *A61B 5/028* (2006.01)
*A61B 5/029* (2006.01)

(86) Internationale Anmeldenummer:
**PCT/EP2017/000214**

(87) Internationale Veröffentlichungsnummer:
**WO 2017/140424 (24.08.2017 Gazette 2017/34)**

(54) **GERÄT ZUR EXTRAKORPORALEN BLUTBEHANDLUNG MIT EINER AUSWERTE- UND STEUEREINHEIT**

EQUIPMENT FOR EXTRA-CORPOREAL TREATMENT OF BLOOD, COMPRISING AN EVALUATION- AND CONTROL UNIT

APPAREIL DE TRAITEMENT EXTRACORPOREL DU SANG COMPRENANT UNE UNITÉ D'ÉVALUATION ET DE COMMANDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.02.2016   DE 102016001710**

(43) Veröffentlichungstag der Anmeldung:
**26.12.2018   Patentblatt 2018/52**

(73) Patentinhaber: **Fresenius Medical Care Deutschland GmbH**
**61352 Bad Homburg (DE)**

(72) Erfinder:
• **MAIERHOFER, Andreas**
**97422 Schweinfurt (DE)**
• **ZHANG, Wei**
**97464 Niederwerrn (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
WO-A1-2009/065611        WO-A1-2011/080194
WO-A2-2011/131358        DE-A1-102013 103 218
DE-C1- 19 702 441          US-A- 6 153 109
US-A1- 2009 054 822

**Beschreibung**

Die Erfindung betrifft ein Blutbehandlungsgerät mit einem extrakorporalen Blutkreislauf, der eine arterielle Leitung, eine Blutpumpe, eine Blutbehandlungseinheit und eine venöse Leitung umfasst, wobei die arterielle und venöse Leitung mit einem Blutgefäß eines Patienten verbunden werden können und wobei das Blutbehandlungsgerät eine Auswerte- und Steuereinheit zur Bestimmung des Blutflusses im beanspruchten Gefäß des Patienten aufweist.

Die Voraussetzung für eine effiziente extrakorporale Blutbehandlung, beispielsweise eine effiziente Dialyse ist das Vorhandensein eines funktionierenden Gefäßzugangs.

Für die extrakorporale Blutbehandlung wird daher oftmals künstlich eine arteriovenöse Fistel hergestellt, meist im Unterarm zwischen der Arteria Radialis und der Vena Cephalica. Durch diesen Kurzschluss von Arterie und Vene wird der Gefäßdruck erhöht, was durch einen verringerten Gewebewiderstand bedingt ist. So wird ein hoher Blutfluss im beanspruchten Gefäß erreicht.

Um einen funktionierenden Gefäßzugang überdies sicherzustellen, werden in einschlägigen Richtlinien verschiedene Methoden empfohlen. Hierzu gehören die Messung der Rezirkulation bei der Entnahme aus dem Gefäßzugang und die Bestimmung des Flusses im Gefäßzugang, wie dies beispielsweise aus Schneditz et al (1998), "Measurement of access flow during hemodialysis using the constant infusion approach", ASAIO journal 44, S. 74ff. bekannt ist. Jedoch kann allein auf Basis von Rezirkulationswerten nur eine sehr grobe Beurteilung des Gefäßzugangs erfolgen. Die Messung des Flusses im Gefäßzugang erfordert eine zeitweise Vertauschung der arteriellen und venösen Nadel, wozu ein spezielles Disposable und Aktionen des Anwenders nötig sind.

Im Stand der Technik ist es beispielsweise aus der WO 2011/131358 A2 bekannt, den Fistelblutfluss auf der Grundlage von Dilutionsmessungen zu bestimmen. Auch die Patente EP 0 773 035 B2 und EP 1 576 341 B1 beschäftigen sich mit der Thematik der Bestimmung des Fistelblutflusses.

Eine Aufgabe der Erfindung ist es, ein Gerät für die extrakorporale Blutbehandlung bereitzustellen, das eine einfache und verlässliche Messung des Blutflusses im Gefäßzugang ermöglicht.

Die Erfindung wird durch die Merkmale des unabhängigen Anspruchs 1 definiert.

Vor diesem Hintergrund betrifft die Erfindung ein Blutbehandlungsgerät mit einem extrakorporalen Blutkreislauf, der eine arterielle Leitung, eine Blutpumpe, eine Blutbehandlungseinheit und eine venöse Leitung umfasst, wobei die arterielle und venöse Leitung mit einem Blutgefäß eines Patienten verbunden werden können und wobei das Blutbehandlungsgerät eine Auswerte- und Steuereinheit aufweist. Erfindungsgemäß ist das Blutbehandlungsgerät dadurch gekennzeichnet, dass die Auswerte- und Steuereinheit ausgebildet ist, um die folgenden Schritte auszuführen: (a1) Ermittlung der Blut-Rezirkulation in einem mit dem extrakorporalen Blutkreislauf verbundenen Blutgefäß des Patienten; und (b) Berechnung des Blutflusses in dem Blutgefäß unter Verwendung der nach (a1) ermittelten Blut-Rezirkulation und eines bereitgestellten oder ebenfalls zuvor ermittelten Wertes für das Herzzeitvolumen des Patienten.

Bei dem Blutbehandlungsgerät kann es sich beispielsweise um ein Dialysegerät oder ein Apheresegerät handeln. Bei der Blutbehandlungseinheit kann es sich beispielsweise um einen Dialysator oder einen Plasmafilter handeln.

In einer Ausführungsform weist das Blutbehandlungsgerät ferner einen Blutdrucksensor auf und die Auswerte- und Steuereinheit ist ausgebildet, um vor Schritt (b) ferner den folgenden Schritt durchzuführen: (a2) Ermittlung des Herzzeitvolumens des Patienten durch Auswertung des zeitlichen Verlaufs eines anhand des Blutdrucksensors gemessenen Druckpulses. Ferner kann der Blutdrucksensor geeignet sein, um den Blutdruck direkt am Patienten zu messen, beispielsweise am Arm oder Handgelenk des Patienten. Geeignete Drucksensoren umfassen piezoelektrische Drucksensoren.

Im Rahmen der Erfindung kann also vorgesehen sein, dass aus oszillometrischen Blutdruckmessungen der Cardiac Output (das Herzzeitvolumen) bestimmt wird. Die Auswertung derartiger oszillometrischer Blutdruckmessungen ist beispielsweise zur Bestimmung des Blutdrucks bekannt (vgl. DE 10 2012 007 081 A1).

Pulsanalysebasierte Verfahren zur Bestimmung des Herzzeitvolumens sind im Stand der Technik bekannt. All diese Verfahren benutzen Pulskurven, um das Herzzeitvolumen zu bestimmen. Die Auswerte- und Steuereinheit des erfindungsgemäßen Blutbehandlungsgeräts kann ausgebildet sein, um das Herzzeitvolumen anhand eines derartigen pulsanalysebasierten Verfahrens zu ermitteln und sodann in Schritt (b) zu verwenden.

Geeignete pulsanalysebasierte Verfahren umfassen die Impulsantwortmethode, die beispielsweise in der US 2011/0034813 A1 genauer beschrieben wird, oder die Modelflussmethode, die in der EP 0 569 506 B1 genauer geschrieben wird.

Bevorzugt ist die Ermittlung des Herzzeitvolumens aus dem mittleren arteriellen Blutdruck, dem zentralvenösen Druck und dem peripheren Widerstand, wie dies beispielsweise im kommerziell verfügbaren Gerät "Vicorder" der Fa. SMT medical GmbH zum Einsatz kommt.

In einer Ausführungsform weist das Blutbehandlungsgerät ferner einen in der arteriellen Leitung des extrakorporalen Blutkreislaufs angeordneten Bolus-Sensor auf und die Auswerte- und Steuereinheit ist ausgebildet, um den Schritt (a1) in der folgenden Weise durchzuführen: (a1) Ermittlung der Blut-Rezirkulation in einem mit dem extrakorporalen Blutkreislauf verbundenen Blutgefäß des Patienten unter Verwendung des Signals des Bolus-Sensors.

In einer Ausführungsform weist das Blutbehandlungsgerät eine Steuereinheit und einen Aktor auf, wobei der Aktor derart ausgebildet ist, dass stromabwärts einer im extrakorporalen Blutkreislauf angeordneten Blutpumpe und/oder der Blutbehandlungseinheit eine Bolusgabe erfolgen kann, und wobei die Steuereinheit so ausgebildet ist, dass unter Verwendung des Aktors während eines Messintervalls ein oder mehrmals eine Bolusgabe erfolgt. Bei dem Aktor kann es sich beispielsweise um eine Heizung handeln, mit der ein Temperaturbolus erzeugt werden kann. Ferner kann es sich bei dem Aktor um ein in den extrakorporalen Blutkreislauf mündendes Zudosiersystem handeln, mit dem ein Konzentrationsbolus oder Temperaturbolus erzeugt werden kann.

Vorzugsweise weist das Blutbehandlungsgerät ferner einen in der venösen Leitung des extrakorporalen Blutkreislaufs angeordneten Bolus-Sensor auf, wobei die Auswerte- und Steuereinheit ausgebildet ist, um die Blut-Rezirkulation im Gefäßabschnitt des Patienten unter Verwendung der Signale des arteriellen und venösen Bolus-Sensors zu bestimmen. Insbesondere kann vorgesehen sein, dass die Auswerte- und Steuereinheit hierbei die Ähnlichkeit der Signalverläufe und den zeitlichen Versatz der von den unterschiedlichen Sensoren erhaltenen Signale berücksichtigt.

Bei dem oder den Bolussensoren kann es sich um Temperatursensoren handeln, um einen Temperaturbolus zu erkennen.

Die Messung der Rezirkulation zwischen venöser und arterieller Nadel mittels Thermodilution wird beispielsweise in Schneditz el al (2003), "Surveillance of Access Function by the Blood Temperature Monitor", Seminars in Dialysis 16, S. 483ff. beschrieben. Hierbei wird blutseitig stromabwärts des Dialysators ein Temperaturbolus erzeugt, der durch Sensorik am venösen und arteriellen Schlauchsystem detektiert wird. Durch den Vergleich der venös und arteriell gemessenen Temperaturboli wird die Rezirkulation berechnet. Die spezifizierte Messgenauigkeit beträgt $\pm 2\%$. Die Interpretation der Werte erfolgt ohne Berücksichtigung von Vitalparametern des Patienten oder Behandlungsparametern. So werden pauschal Werte <10% als normale cardiopulmonare Rezirkulation betrachtet, während größere Werte als Anzeichen für das Vorliegen einer Rezirkulation im Gefäßzugang, z.B. durch Vertauschung von arterieller und venöser Nadel, gewertet werden.

In einer Ausführungsform ist die Auswerte- und Steuereinheit ausgebildet, um bei der Ermittlung des Blutflusses in Schritt (b) neben der Blut-Rezirkulation und dem Herzzeitvolumen ferner den extrakorporalen Blutfluss und den Abfluss von Flüssigkeit in der Blutbehandlungseinrichtung zu berücksichtigen. Der extrakorporale Blutfluss $Q_b$ kann beispielsweise durch Einstellung der Förderrate einer in der arteriellen Leitung vorhandenen Blutpumpe eingestellt werden. Bei dem Abfluss von Flüssigkeit in der Blutbehandlungseinrichtung kann es sich insbesondere um die Ultrafiltrationsrate handeln. Diese kann beispielsweise anhand einer UF-Pumpe eingestellt werden, die in einem ebenfalls an den Dialysator angeschlossenen Dialysierflüssigkeitssystem angeordnet ist.

In einer Ausführungsform ist die Auswerte- und Steuereinheit ausgebildet, um unter der Annahme, dass der Blutfluss im betroffenen Gefäß maximal einen bestimmten Anteil des Herzzeitvolumens erreichen kann, kritische Werte für die Rezirkulation für einen normalen und/oder inversen Anschluss der arteriellen und venösen Leitung am Blutgefäß zu definieren. Der maximale Anteil am Herzzeitvolumen, den der Blutfluss im betroffenen Gefäß maximal erreichen kann, kann beispielsweise bei 50% liegen. So können kritische Werte für die Rezirkulation bei normaler und inverser Nadelposition definiert werden. Die kritischen Werte unterscheiden sich je nach Fluss im extrakorporalen Blutkreislauf.

In einer Ausführungsform ist die Auswerte- und Steuereinheit ausgebildet, um die ermittelte Rezirkulation mit diesen kritischen Werten zu vergleichen und auf der Grundlage des Vergleichs zu gruppieren.

In einer Ausführungsform weist das Gerät ferner eine Ausgabeeinheit auf, die mit der Auswerte- und Steuereinheit in Verbindung steht, wobei die Ausgabeeinheit und die Auswerte- und Steuereinheit so ausgebildet sind, um je nach Gruppenzugehörigkeit der ermittelten Rezirkulation ein unterschiedliches Signal an den Benutzer auszugeben. Die Ausgabeeinheit kann beispielsweise akustische und/oder visuelle Signale an den Benutzer ausgeben.

In einer Ausführungsform werden drei Gruppen unterschieden: (1) die ermittelte Rezirkulation liegt unterhalb des kritischen Werts für normalen Anschluss; (2) die ermittelte Rezirkulation liegt oberhalb des kritischen Werts für normalen Anschluss aber unterhalb des kritischen Werts für inversen Anschluss; und (3) die ermittelte Rezirkulation liegt oberhalb des kritischen Werts für inversen Anschluss.

Hinsichtlich der Signalausgabe kann beispielsweise dann, wenn die ermittelte Rezirkulation der Gruppe (1) zugeordnet werden kann, ein Signal ausgegeben werden, das auf einen korrekten Anschluss des extrakorporalen Blutkreislaufs am Gefäß hinweist, und/oder dann, wenn die ermittelte Rezirkulation der Gruppe (3) zugeordnet werden kann, ein Signal ausgegeben werden, das auf eine Vertauschung der arteriellen und venösen Anschlüsse hinweist.

In einer Ausführungsform ist die Auswerte- und Steuereinheit so ausgebildet, dass dann, wenn die ermittelte Rezirkulation der Gruppe (2) zugeordnet wurde, die Förderleistung der Blutpumpe verringert wird und die Durchführung der Schritte (a1) und (b) wiederholt wird.

In einer Ausführungsform ist die Auswerte- und Steuereinheit ausgebildet, um ein Warnsignal auszugeben, wenn der ermittelte Blutfluss im betroffenen Gefäß einen oberen Schwellenwert übersteigt oder einen unteren Schwellenwert unterschreitet.

In diesem Fall kann eine Überprüfung des erfindungsgemäß ermittelten Wertes durch Vertauschen der arteriellen und venösen Nadeln und Anwendung eines vorbekannten Verfahrens angezeigt sein. Der untere Schwellenwert kann beispielsweise bei 300 oder 500 ml/min liegen und der obere Schwellenwert beispielsweise bei 2000 oder 1500 ml/min.

Die Auswerte- und Steuereinheit kann vorzugsweise die für die Datensammlung notwendigen Eingriffe im Dialysegerät (z.B. Betrieb der Blutpumpe bei einer bestimmten Rate oder Gabe eines Temperaturbolus) einleiten. Sie kann so ausgebildet sein, dass die ermittelten Daten oder Schlussfolgerungen (z.B. der Blutfluss im betroffenen Gefäß, korrekter oder inverser Anschluss der Nadeln, Signale hinsichtlich eines korrekten Zugangs, etc.) automatisch zur Steuerung des Gerätes verwendet werden.

In einer Ausführungsform weist das Blutbehandlungsgerät eine akustische und/oder visuelle Ausgabeeinheit auf, mit welcher die von der Auswerte- und Steuereinheit ermittelten Daten oder Schlussfolgerungen (s.o.) an einen Benutzer ausgibt.

Ferner umfasst die Erfindung ein Verfahren zur Bestimmung des Blutflusses in einem mit dem extrakorporalen Blutkreislauf einer vorzugsweise erfindungsgemäßen Blutbehandlungseinrichtung verbundenen Blutgefäß eines Patienten. Dabei ist vorgesehen, die Rezirkulation im Gefäß zu bestimmen und unter Zugrundelegung dieser Rezirkulation sowie eines ebenfalls bestimmten oder anderweitig abgeschätzten Wertes für das Herzzeitvolumen den Blutfluss im betroffenen Blutgefäß des Patienten zu bestimmen.

Neben der eingangs genannten Aufgabe ist es ferner eine Aufgabe der Erfindung, ein Gerät für die extrakorporale Blutbehandlung bereitzustellen, das eine Feststellung der Qualität des Gefäßzugangs ermöglicht.

In diesem Zusammenhang betrifft die Erfindung ein Blutbehandlungsgerät mit einem extrakorporalen Blutkreislauf, der eine arterielle Leitung, eine Blutpumpe, eine Blutbehandlungseinheit und eine venöse Leitung umfasst, wobei die arterielle und venöse Leitung mit einem Blutgefäß eines Patienten verbunden werden können und wobei das Blutbehandlungsgerät eine Auswerte- und Steuereinheit aufweist. Erfindungsgemäß ist vorgesehen, dass die Auswerte- und Steuereinheit ausgebildet ist, um die Blut-Rezirkulation in einem mit dem extrakorporalen Blutkreislauf verbundenen Blutgefäß des Patienten zu ermitteln und die ermittelte Rezirkulation mit ebenfalls ermittelten oder vorgegebenen kritischen Werten zu vergleichen und auf der Grundlage des Vergleichs zu gruppieren. Hier erfolgt eine Gruppierung der Rezirkulationswerte also gegebenenfalls ohne Ermittlung des Blutflusses im Blutgefäß.

Die Ermittlung der Blut-Rezirkulation kann dabei wie oben beschrieben erfolgen.

Auch hier kann das Gerät ferner eine Ausgabeeinheit aufweisen, die mit der Auswerte- und Steuereinheit in Verbindung steht, wobei die Ausgabeeinheit und die Auswerte- und Steuereinheit so ausgebildet sind, um je nach Gruppenzugehörigkeit der ermittelten Rezirkulation ein unterschiedliches Signal an den Benutzer auszugeben. Die Ausgabe kann dabei wie oben beschrieben erfolgen.

Alternativ oder zusätzlich zur Ausgabe an den Benutzer kann eine Reaktion des Blutbehandlungsgeräts initiiert werden.

Analog der ersten Ausgestaltung der Erfindung können auch hier drei Gruppen unterschieden werden: (1) die ermittelte Rezirkulation liegt unterhalb des kritischen Werts für normalen Anschluss; (2) die ermittelte Rezirkulation liegt oberhalb des kritischen Werts für normalen Anschluss aber unterhalb des kritischen Werts für inversen Anschluss; und (3) die ermittelte Rezirkulation liegt oberhalb des kritischen Werts für inversen Anschluss.

Ebenfalls analog der ersten Ausgestaltung der Erfindung kann die Auswerte- und Steuereinheit so ausgebildet sein, dass dann, wenn die ermittelte Rezirkulation der Gruppe (2) zugeordnet wurde, die Förderleistung der Blutpumpe verringert wird und die Ermittlung der Blut-Rezirkulation wiederholt wird.

Letztlich umfasst die Erfindung ein Verfahren zur Bestimmung der Qualität des Gefäßzugangs in einem mit dem extrakorporalen Blutkreislauf einer vorzugsweise erfindungsgemäßen Blutbehandlungseinrichtung verbundenen Blutgefäß eines Patienten. Dabei ist vorgesehen, die Rezirkulation im Gefäß zu bestimmen, die ermittelte Rezirkulation mit ebenfalls ermittelten oder vorgegebenen kritischen Werten zu vergleichen und auf der Grundlage des Vergleichs zu gruppieren.

Weitere Einzelheiten und Vorteile der Erfindung ergeben sich aus dem nachfolgend anhand der Figuren dargestellten Ausführungsbeispiel. In den Figuren zeigen:

Figur 1: eine schematische Darstellung einer Ausführungsform eines erfindungsgemäßen Dialysegeräts;

Figur 2: eine Auftragung von erfindungsgemäß ermittelten kritischen Werten für den Anteil der Rezirkulationen $R_n$ und $R_x$ in normaler bzw. inverser Nadelposition in Abhängigkeit des Herzzeitvolumens CO für extrakorporale Blutflüsse von 200, 300 und 400 ml/min;

Figur 3: eine Auftragung des relativen Fehlers bei der erfindungsgemäßen Bestimmung des Shuntflusses $Q_a$ bei einem Blutfluss von 300 ml/min für Werte von $Q_a$ von 400 bis 2000 ml/min;

Figur 4: eine Auftragung des Shuntflusses $Q_a$ in Abhängigkeit von CO bei verschiedenen Werten der Rezirkulation $R_x$ in inverser Nadelpostition bei einem extrakorporalen Blutfluss von 300 ml/min; und

Figur 5:    eine Auftragung des relativen Fehlers bei der Bestimmung des Shuntflusses $Q_a$ bei vertauschten Nadeln und einem extrakorporalen Blutfluss von 300 ml/min.

Theoretischer Hintergrund:

Der in der Steuereinheit des erfindungsgemäßen Dialysegeräts gemäß Ausführungsbeispiel hinterlegte Algorithmus basiert auf dem nachfolgend erläuterten theoretischen Hintergrund.

Die cardiopulmonare Rezirkulation bei Hämodialysepatienten mit einem Herzzeitvolumen CO, die über einen Gefäßzugang mit einem Shuntfluss $Q_a$ behandelt werden, ist definiert als

$$CPR = \frac{Q_a}{CO}$$

**Formel 1**

Gemäß Schneditz (1998) kann durch Messung des Anteils der Rezirkulationen $R_n$ und $R_x$ in normaler bzw. inverser Nadelpostition der Shuntfluss $Q_a$ bei bekanntem extrakorporalem Blutfluss $Q_b$ und UF-Rate $O_f$ bestimmt werden. Hierfür werden folgende Größen definiert.

$$f_n = \frac{R_n}{1-R_n} \, , \, f_x = \frac{R_x}{1-R_x} \, , \, \tilde{Q} = Q_b - Q_f$$

**Formel 2**

Hiermit lautet die von Schneditz (1998) angegebene Formel.

$$Q_a = \frac{1}{f_x} \frac{\tilde{Q}}{1-CPR} \text{ mit } CPR = \frac{f_n}{f_x} \frac{\tilde{Q}}{Q_b}$$

**Formel 3**

Ist der Cardiac Output bekannt, so kann $Q_a$ bei Kenntnis von nur einer der Größen $R_n$ oder $R_x$ berechnet werden.

$$Q_a = CO - \frac{\tilde{Q}}{f_n}$$

**Formel 4**

$$Q_a = \frac{CO}{2} - \sqrt{\left(\frac{CO}{2}\right)^2 - \frac{\tilde{Q}^2}{Q_b} \frac{CO}{f_x}}$$

**Formel 5**

Nach dem Fehlerfortpflanzungsgesetz lässt sich der Fehler der Bestimmung von $Q_a$ aus CO und $R_n$ wie folgt abschätzen.

$$\sigma_{Q_a} = \sqrt{\sigma_{CO}^2 + \frac{(1-R_n)^2}{R_n^2}\sigma_{\tilde{Q}}^2 + \left(\frac{\tilde{Q}}{R_n^2}\right)^2 \sigma_{R_n}^2}$$

Formel 6

$$\sigma_{Q_a} = \frac{1}{2}\sqrt{\left[1 - \frac{\frac{1}{2}CO - \frac{(1-R_x)\tilde{Q}}{R_x}}{W}\right]\sigma_{CO}^2 + \left(\frac{(1-R_x)CO}{R_x W}\right)^2 \sigma_{\tilde{Q}}^2 + \left(\frac{\tilde{Q}CO}{R_x^2 W}\right)^2 \sigma_{R_x}^2}$$

$$\text{mit } W = \sqrt{\left(\frac{CO}{2}\right)^2 - \frac{\tilde{Q}CO}{f_x}}$$

Formel 7

Aus Formel 4 bzw. Formel 5 folgt.

$$R_n = \frac{\tilde{Q}}{\tilde{Q} + CO - Q_a} \; , \; R_x = \frac{\frac{\tilde{Q}^2}{Q_b}}{\frac{\tilde{Q}^2}{Q_b} + Q_a - \frac{Q_a^2}{CO}}$$

Formel 8

Unter der physiologisch begründeten Annahme, dass der Shuntfluss $Q_a$ maximal 50% des Cardiac Output CO erreichen kann, ergeben sich für $R_n$ und $R_x$ kritische Werte, die bei einer Messung der Rezirkulation nicht über bzw. unterschritten werden können.

$$R_{n,crit} = \frac{1}{1 + \frac{1}{2}\frac{CO}{\tilde{Q}}} \; , \; R_{x,crit} = \frac{1}{1 + \frac{1}{4}\frac{Q_b}{\tilde{Q}^2}CO}$$

Formel 9

Die Messung des Shunt-Flusses ist aus verschiedenen Gründen gewünscht. Neben niedrigen sind auch gefährlich hohe Shunt-Flüsse wichtig zu erkennen (Steal-Syndrom). Gemäß der vorliegenden Idee berechnet sich der Shuntfluss $Q_a$ also zu $Q_a = CO - Q_b/f_n$ mit $f_n = R_n/1 - R_n$. Dabei ist R die Rezirkulationsfraktion und $Q_b$ der extrakorporale Blutfluss. Das Herzminutenvolumen CO und die Rezirkulation werden experimentell bestimmt. Somit lassen sich ohne Disposables und ohne Eingriff des Anwenders gefährlich hohe Shunt-Flüsse erkennen. Das Konzept kommt in einer Ausführungsform auch ohne die Einführung eines Bolus aus. Bei vertauschten Nadeln ist wegen der höheren Rezirkulation eine genauere Bestimmung auch bei niedrigen Shunt-Flüssen denkbar.

Die Ermittlung des Herzzeitvolumens kann beispielsweise anhand der Formel

$$CO = (MAP-CVP)/R_P$$

erfolgen, wobei MAP der mittlere arterielle Blutdruck, CVP der zentralvenöse Druck und Rp der periphere Widerstand ist. Diese nach dem Prinzip "Fluss=Druck/Widerstand" arbeitende Methode kommt beispielsweise in den kommerziell verfügbaren Geräten Vicorder der Fa. SMT medical GmbH zum Einsatz. Der zentralvenöse Druck kann dabei beispielsweise anhand eines zentralvenösen Katheters gemessen oder abgeschätzt und manuell eingegeben werden und der periphere Widerstand aus der fallenden Flanke einer Pulskurve bestimmt werden.

Ausführungsbeispiel:

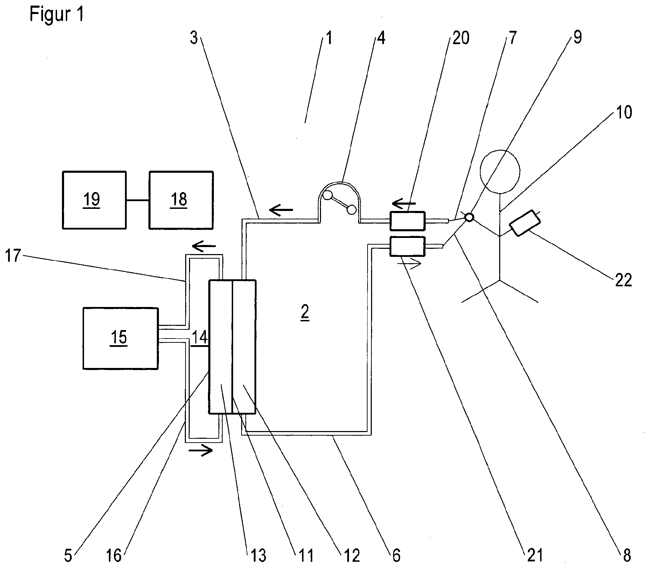

Eine schematische Darstellung einer Ausführungsform eines erfindungsgemäßen Dialysegeräts ist in Figur 1 gezeigt.

Das Dialysegerät ist in der Figur allgemein mit dem Bezugszeichen 1 gekennzeichnet. Es weist einen extrakorporalen Blutkreislauf 2 auf, der in bekannter Weise eine arterielle Leitung 3 mit einer Blutpumpe 4, einen Dialysator 5 und eine venöse Leitung 6 umfasst. Die arterielle Leitung 3 und die venöse Leitung 6 sind anhand einer arteriellen Nadel 7 bzw. einer venösen Nadel 8 mit einem Gefäß 9 eines Patienten 10 verbunden.

Im Dialysator 5 ist eine semipermeable Membran 11 angeordnet, die innerhalb des Dialysators 5 die Blutkammer 12 von der Dialysierflüssigkeitskammer 13 trennt. An der Blutkammer 12 sind die arterielle und venöse Leitung 3 bzw. 6 des extrakorporalen Blutkreislaufs 2 angeschlossen. An der Dialysierflüssigkeitskammer 13 ist ein Dialysierflüssigkeitssystem 14 angeschlossen, welches eine Vorrichtung 15 zur Aufbereitung von Dialysierflüssigkeit, eine Zuleitung 16 zum Dialysator 5 und eine Ableitung 17 vom Dialysator 5 umfasst. In der Ableitung 17 kann eine in der Figur nicht dargestellte Ultrafiltrationspumpe angeordnet sein.

Die Flussrichtungen des Blutes im extrakorporalen Blutkreislauf 2 und der Dialsysierflüssigkeit im Dialysierflüssigkeitssystem 14 sind in der Figur anhand von Pfeilen dargestellt.

Ferner umfasst das Dialysegerät 1 eine Auswerte- und Steuereinheit 18 sowie eine Ausgabeeinheit 19.

Sowohl an der arteriellen Leitung 3 als auch an der venösen Leitung 6 sind nahe der jeweiligen Nadeln Temperatursensoren 20 bzw. 21 angeordnet.

Das Gerät 1 umfasst ferner in der Figur nicht dargestellt Mittel zur Änderung der Bluttemperatur in der venösen Blutleitung. Diese Mittel können beispielsweise darin bestehen, dass die Temperatur der in der Vorrichtung 15 erzeugten Dialysierflüssigkeit nach Vorgabe der Auswerte- und Steuereinheit 18 mit dem Ziel einer Änderung der Bluttemperatur variiert werden. Alternativ kann die Änderung der Bluttemperatur z.B. auch durch am Blutschlauchsystem angebrachte Peltierelemente erfolgen.

Letztlich umfasst das Gerät 1 einen Sensor 22, um einen Pulsdruckkurvenverlauf des Patienten zu messen, der geeignet ist, CO zu bestimmen, z.B. durch einen Manschette am Oberarm. Der Sensor ist in einer in der Figur nicht dargestellten Weise mit der Auswerte- und Steuereinheit 18 verbunden.

Im Betrieb der Vorrichtung saugt die Blutpumpe 4 über die arterielle Nadel 7 Blut aus dem Gefäß 9 des Patienten 10 in die arterielle Leitung 3 des extrakorporalen Blutkreislaufs 2 und pumpt das Blut anschließend durch den Dialysator 5, die venöse Leitung 6 und die venöse Nadel 8 zurück in das Gefäß 9 des Patienten 10. Nach Gabe eines Temperaturbolus wird der zeitliche Temperaturverlauf des entnommenen und zurückgegebenen Blutes an den Sensoren 20 und 21 gemessen und die Messwerte werden an die Auswerte- und Steuereinheit 18 übermittelt. Aus dem Temperaturverlauf wird dann in der Auswerte- und Steuereinheit 18 die Rezirkulation R wie in Schneditz (2003) beschrieben bestimmt. Ferner wird mittels oszillometrischer Blutdruckmessungen am Blutdrucksensor das Herzzeitvolumen bestimmt.

Alternativ kann der Cardiac Output bei bekanntem, beispielsweise aus echocardiographischen Untersuchungen stammendem oder als typischer Wert von 70 ml abgeschätztem Herz-Schlagvolumen $V_{CO}$, durch Messung der Herzrate v, mittel $CO = v \cdot V_{CO}$ abgeschätzt werden.

Nach Bestimmung von R und CO und bei bekannter Förderrate der Blutpumpe 4 und Ultrafiltrationsrate werden in der Auswerte- und Steuereinheit 18 nun die oben näher dargestellten Berechnungen zur Ermittlung des Blutflusses im Gefäß 9 des Patienten 10 durchgeführt. Die Ergebnisse können beispielsweise an der Ausgabeeinheit 19 ausgegeben werden, über eine beliebige Art der Kommunikation wie beispielsweise über Netzwerk übermittelt werden und/oder automatisch für die Steuerung des Gerätes 1 verwendet werden.

Interpretation und Nutzung der Ergebnisse:

Die Ergebnisse können in der nachfolgend beschriebenen Weise interpretiert bzw. genützt werden.

Wird zeitnah zur Messung der Rezirkulation R das Herzzeitvolumen CO gemessen oder abgeschätzt, können zusammen mit den bekannten Werten für den extrakorporalen Blutfluss und UF Rate gemäß Formel 9 kritische Werte

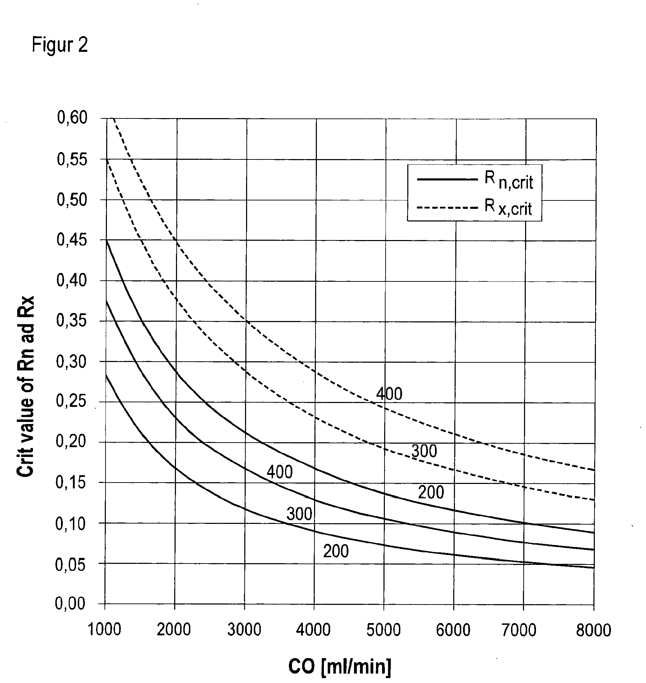

$R_{n,crit}$ und $R_{x,crit}$ für die Rezirkulationen $R_n$ und $R_x$ in normaler bzw. inverser Nadelposition berechnet und R mit diesen Werten verglichen werden. Figur 2 zeigt die nach Formel 9 berechneten kritischen Werte für $R_n$ und $R_x$ in Abhängigkeit des Herzzeitvolumens CO für extrakorporale Blutflüsse von 200, 300 und 400 ml/min.

Die kritischen Werte können in einer weiteren Ausgestaltung der Erfindung aber auch anderweitig bestimmt oder vorgegeben werden.

Es lassen sich unterschiedliche Fälle unterscheiden, die unterschiedliche Schlussfolgerungen zulassen.

Ist $R < R_{n,crit}$, so liegt nur cardiopulmonare Rezirkulation vor. Der Shuntfluss ist daher größer als der extrakorporale Blutfluss, arterielle und venöse Nadel sind korrekt punktiert und an das Schlauchsystem konnektiert. Hierüber kann der Anwender mittels der Ausgabeeinheit 19 auf beliebige Weise informiert werden.

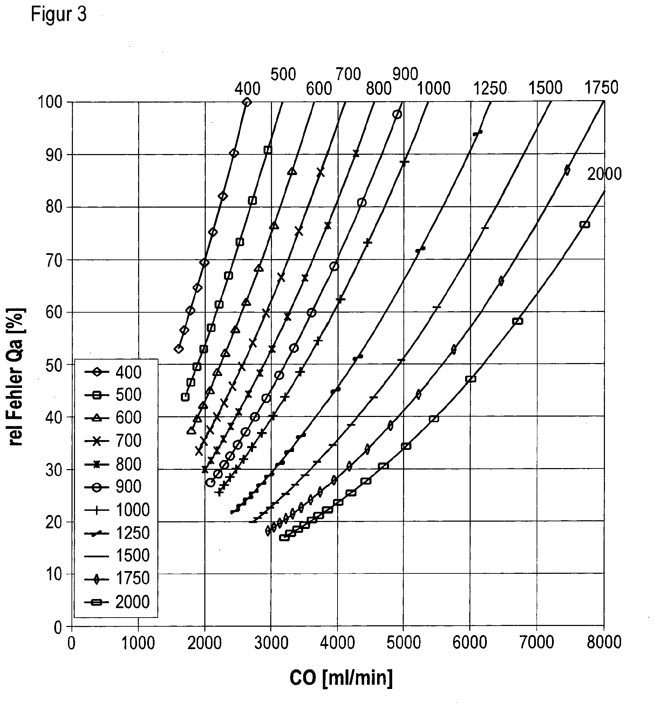

Ferner kann mittels Formel 4 der Shuntfluss $Q_a$ abgeschätzt werden. Figur 3 zeigt den nach Formel 6 berechneten relativen Fehler bei der Bestimmung von $Q_a$ bei einem Blutfluss von 300 ml/min unter Annahme eines Fehlers der Rezirkulationsmessung von $\pm 1\%$ und der Bestimmung von CO und von $Q_b$ von $\pm 10\%$ für Werte von $Q_a$ von 400 bis 2000 ml/min. Hieraus wird deutlich, dass bei niedrigen $Q_a$ die Messgenauigkeit stark eingeschränkt ist. Dennoch ist sichergestellt, dass auf jeden Fall $Q_a > Q_b$ ist. Da der relative Fehler für hohe Shuntflüsse sinkt, kann die Bestimmung von $Q_a$ dazu verwendet werden, um gefährlich hohe Shuntflüsse erkennen zu können. Shuntflüsse > 2000 ml/min belasten das Herz des Patienten, was zu einer erhöhten Mortalität führt. Zugleich führen sie zu Mangeldurchblutungen der distal des (typischerweise nahe des Handgelenks liegenden) Gefäßzugangs liegenden Gliedmaßen, d.h. besonders von Hand und Fingern, was zu deren Schädigung und zu Beeinträchtigungen des Patienten führen kann. Daher kann basierend auf einem aus $R_n$ und CO bestimmten Shuntfluss eine Warnung vor zu hohem Shuntfluss generiert werden. Wird ein zu niedriger Shuntfluss von < 600 ml/min oder ein zu hoher Shuntfluss mit der beschriebenen Methode detektiert, so kann der Anwender aufgefordert werden, nun zur genaueren Bestimmung des Shuntflusses die Nadeln zu vertauschen, was vorteilhafterweise dann leicht möglich ist, wenn bereits ein Disposable hierzu vorhanden ist. Aus der Bestimmung von $R_x$ kann dann $Q_a$ gemäß Formel 3 genauer bestimmt werden, die Bestimmung aus $R_n$ und CO würde dann nur als Vorscreening dienen.

Ist $R > R_{x,crit}$, so ist die Gefäß-Rezirkulation so hoch, dass mit hoher Wahrscheinlichkeit eine Vertauschung von arterieller und venöser Nadel vorliegt. Auch hierüber kann der Anwender mittels der Ausgabeeinheit 19 auf beliebige Weise informiert werden.

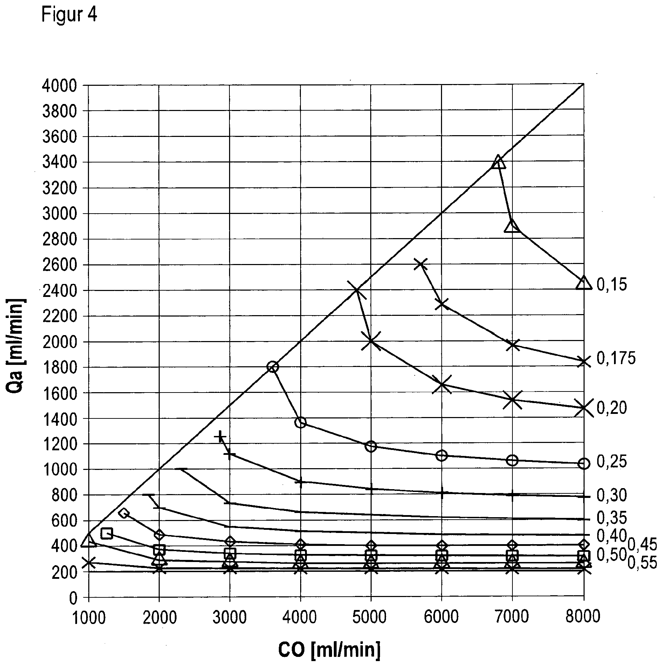

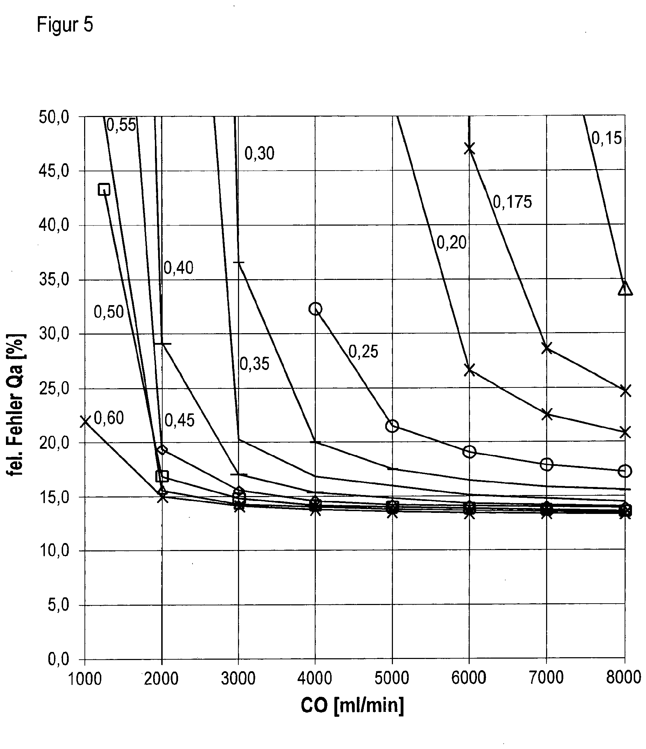

Figur 4 zeigt eine Auftragung des Shuntflusses $Q_a$ in Abhängigkeit von CO bei verschiedenen Werten von $R_x$ nach Formel 5 bei einem extrakorporalen Blutfluss von 300 ml/min. Daraus lässt sich erkennen, dass sich gerade bei niedrigen Shuntflüssen (< 800 ml/min) der Shuntfluss allein aus der gemessenen Rezirkulation $R_x$ bei vertauschten Nadeln bestimmen lässt und dass der Wert des CO nur geringfügig den Wert des Shuntflusses beeinflusst, wenn $Q_a <$ ¼ CO ist. Dieses ist auch in Figur 5 zu erkennen, wo der relative Fehler bei der Bestimmung von $Q_a$ nach Formel 7 bei einem extrakorporalen Blutfluss von 300 ml/min aufgetragen wurde. Somit kann schon bei nur ungenauer Kenntnis von CO der Shuntfluss allein durch Messung der Rezirkulation bei vertauschten Nadeln bestimmt werden, ohne eine Rezirkulationsmessung in normaler Nadelorientierung zu benötigen. Damit kann die zur Bestimmung des Shuntflusses benötigte Zeit deutlich reduziert werden. Die Nadelvertauschung muss im Allgemeinen manuell durch den Benutzer unter Verwendung eines entsprechenden Disposables erfolgen. Für den Fall einer bereits fälschlicherweise vorliegenden Nadelvertauschung kann, nachdem eine Nadelvertauschung aus der Messung der Rezirkulation und des CO erkannt wurde, sofort mittels Formel 5 der Shuntfluss angegeben werden.

Ist $R_{n,crit} < R < R_{x,crit}$, so liegt eine teilweise Rezirkulation zwischen arterieller und venöser Nadel vor, was durch eine ungünstige Positionierung der Nadeln (z.B. zu dicht nebeneinander) entstehen kann. Alternativ kann der extrakorporale Blutfluss den Shuntfluss überschreiten. Es kann vorgesehen sein, dass die Auswerte- und Steuereinheit 18 zur Unterscheidung dieser beiden Szenarien so ausgebildet ist, dass automatisch eine Rezirkulationsmessung bei reduziertem Blutfluss $Q_b'$ durchgeführt wird und die Werte erneut ermittelt werden. Liegt der dann bestimmte Wert R' unterhalb von $R'_{n,crit}$, so liegt $Q_a$ zwischen $Q_b'$ und $Q_b$.

Zusammenfassend ergibt sich, dass die vorliegende Erfindung eine Möglichkeit zur Erkennung eines vor allem übermäßig hohen Shuntflusses an die Hand gibt. Ein sehr hoher Shuntfluss ist medizinisch unerwünscht. Gemäß der Erfindung wird beispielsweise anhand Gabe eines Temperaturbolus die Rezirkulation und beispielsweise anhand einer oszillometrischen Blutdruckmessung das Herzzeitvolumen bestimmt. Aus beiden wird anhand der Formel 4 der Shuntfluss berechnet. Dieser lässt sich vor allem bei sehr hohen Flüssen mit relativ geringem Fehler bestimmen. Ferner erlaubt die Erfindung die Bestimmung zweier Grenzwerte der Rezirkulation und die Ableitung entsprechender Schlussfolgerungen. Ein weiterer Aspekt der Erfindung beschäftigt sich insbesondere mit dem Vergleich der Rezirkulation mit Grenzwerten und der Ableitung von Schlussfolgerungen, losgelöst davon, wie die Grenzwerte ermittelt werden.

**Patentansprüche**

**1.** Blutbehandlungsgerät (1) mit einem extrakorporalen Blutkreislauf, der eine arterielle Leitung, eine Blutpumpe, eine Blutbehandlungseinheit und eine venöse Leitung umfasst, wobei die arterielle und venöse Leitung mit einem Blutgefäß eines Patienten verbunden werden können und wobei das Blutbehandlungsgerät eine Auswerte- und Steuereinheit aufweist,

wobei die Auswerte- und Steuereinheit ausgebildet ist, um die folgenden Schritte auszuführen:

(a1) Ermittlung der Blut-Rezirkulation in einem mit dem extrakorporalen Blutkreislauf verbundenen Blutgefäß des Patienten; und

(b) Berechnung des Blutflusses in dem Blutgefäß unter Verwendung der nach (a1) ermittelten Blut-Rezirkulation und eines bereitgestellten oder ebenfalls zuvor ermittelten Wertes für das Herzzeitvolumen des Patienten

und **gekennzeichnet dadurch, dass** die Auswerte- und Steuereinheit ausgebildet ist, um bei der Ermittlung des Blutflusses in Schritt (b) neben der Blut-Rezirkulation und dem Herzzeitvolumen ferner den extrakorporalen Blutfluss und den Abfluss von Flüssigkeit in der Blutbehandlungseinrichtung zu berücksichtigen..

2. Blutbehandlungsgerät nach Anspruch 1, **dadurch gekennzeichnet, dass** das Blutbehandlungsgerät ferner einen Blutdrucksensor aufweist und dass die Auswerte- und Steuereinheit ausgebildet ist, um ferner den folgenden Schritt durchzuführen:

(a2) Ermittlung des Herzzeitvolumens des Patienten durch Auswertung des zeitlichen Verlaufs eines anhand des Blutdrucksensors gemessenen Druckpulses.

3. Blutbehandlungsgerät nach einem der vorhergehenden Ansprüche, **dadurch gekennzeichnet, dass** das Blutbehandlungsgerät ferner einen in der arteriellen Leitung des extrakorporalen Blutkreislaufs angeordneten Bolus-Sensor aufweist und dass die Auswerte- und Steuereinheit ausgebildet ist, um den Schritt (a1) in der folgenden Weise durchzuführen:

(a1) Ermittlung der Blut-Rezirkulation in einem mit dem extrakorporalen Blutkreislauf verbundenen Blutgefäß des Patienten unter Verwendung des Signals des Bolus-Sensors.

4. Blutbehandlungsgerät nach einem der vorhergehenden Ansprüche, **dadurch gekennzeichnet, dass** die Auswerte- und Steuereinheit ausgebildet ist, um unter der Annahme, dass der Blutfluss im betroffenen Gefäß maximal einen bestimmten Anteil des Herzzeitvolumens erreichen kann, kritische Werte für die Rezirkulation für einen normalen und/oder inversen Anschluss der arteriellen und venösen Leitung am Blutgefäß zu definieren.

5. Blutbehandlungsgerät nach Anspruch 4, **dadurch gekennzeichnet, dass** die Auswerte- und Steuereinheit ausgebildet ist, um die ermittelte Rezirkulation mit diesen kritischen Werten zu vergleichen und auf der Grundlage des Vergleichs zu gruppieren.

6. Blutbehandlungsgerät nach Anspruch 5, **dadurch gekennzeichnet, dass** das Gerät ferner eine Ausgabeeinheit aufweist, die mit der Auswerte- und Steuereinheit in Verbindung steht, wobei die Ausgabeeinheit und die Auswerte- und Steuereinheit so ausgebildet sind, um je nach Gruppenzugehörigkeit der ermittelten Rezirkulation ein unterschiedliches Signal an den Benutzer auszugeben.

7. Blutbehandlungsgerät nach Anspruch 5 oder 6, **dadurch gekennzeichnet, dass** drei Gruppen unterschieden werden:

(1) die ermittelte Rezirkulation liegt unterhalb des kritischen Werts für normalen Anschluss;
(2) die ermittelte Rezirkulation liegt oberhalb des kritischen Werts für normalen Anschluss aber unterhalb des kritischen Werts für inversen Anschluss; und
(3) die ermittelte Rezirkulation liegt oberhalb des kritischen Werts für inversen Anschluss.

8. Blutbehandlungsgerät nach Anspruch 7, **dadurch gekennzeichnet, dass** die Auswerte- und Steuereinheit so ausgebildet ist, dass dann, wenn die ermittelte Rezirkulation der Gruppe (2) zugeordnet wurde, die Förderleistung der Blutpumpe verringert wird und die Durchführung der Schritte (a1) und (b) wiederholt wird.

9. Blutbehandlungsgerät nach einem der vorhergehenden Ansprüche, **dadurch gekennzeichnet, dass** die Auswerte- und Steuereinheit ausgebildet ist, um ein Warnsignal auszugeben, wenn der ermittelte Blutfluss im betroffenen Gefäß einen oberen Schwellenwert übersteigt oder einen unteren Schwellenwert unterschreitet.

## EP 3 416 701 B1

**Claims**

1. A blood treatment device (1) having an extracorporeal blood circuit which comprises an arterial line, a blood pump, a blood treatment unit and a venous line, wherein the arterial and venous lines can be connected to a blood vessel of a patient, and wherein the blood treatment device has an evaluation and control unit,
wherein the evaluation and control unit is configured to carry out the following steps:

   (a1) determining the blood recirculation in a blood vessel of the patient connected to the extracorporeal blood circuit; and
   (b) calculating the blood flow in the blood vessel using the blood recirculation determined in accordance with (a1) and using a provided value or a value likewise previously determined for the cardiac output of the patient

   and **characterized in that**
   the evaluation and control unit is configured furthermore to take account of the extracorporeal blood flow and the outflow of fluid in the blood treatment apparatus, in addition to the blood recirculation and the cardiac output, when determining the blood flow in step (b).

2. A blood treatment device in accordance with claim 1, **characterized in that** the blood treatment device furthermore has a blood pressure sensor; and **in that** the evaluation and control unit is configured furthermore to carry out the following step:
   (a2) determining the cardiac output of the patient by evaluating the time progression of a pressure pulse measured using the blood pressure sensor.

3. A blood treatment device in accordance with one of the preceding claims, **characterized in that** the blood treatment device furthermore has a bolus sensor arranged in the arterial line of the extracorporeal blood circuit; and **in that** the evaluation and control unit is configured to carry out step (a1) in the following manner:
   (a1) determining the blood recirculation in a blood vessel of the patient connected to the extracorporeal blood circuit using the signal of the bolus sensor.

4. A blood treatment device in accordance with one of the preceding claims, **characterized in that** the evaluation and control unit is configured to define critical values for the recirculation for a normal and/or inverse connection of the arterial and venous lines to the blood vessel under the assumption that the blood flow in the corresponding vessel can achieve a specific portion of the cardiac output as a maximum.

5. A blood treatment device in accordance with claim 4, **characterized in that** the evaluation and control unit is configured to compare the determined recirculation with these critical values and to group it on the basis of the comparison.

6. A blood treatment device in accordance with claim 5, **characterized in that** the device furthermore has an output unit which communicates with the evaluation and control unit, wherein the output unit and the evaluation and control unit are configured to output a different signal to the user depending on the group association of the determined recirculation.

7. A blood treatment device in accordance with claim 5 or claim 6, **characterized in that** three groups are distinguished:

   (1) the determined recirculation is below the critical value for a normal connection;
   (2) the determined recirculation is above the critical value for a normal connection, but below the critical value for an inverse connection; and
   (3) the determined recirculation is above the critical value for an inverse connection.

8. A blood treatment device in accordance with claim 7, **characterized in that** the evaluation and control unit is configured such that, when the determined recirculation has been associated with group (2), the conveying performance of the blood pump is reduced and the carrying out of steps (a1) and (b) is repeated.

9. A blood treatment device in accordance with one of the preceding claims, **characterized in that** the evaluation and control unit is configured to output a warning signal when the determined blood flow in the respective vessel exceeds an upper threshold value or falls below a lower threshold value.

**Revendications**

1. Appareil de traitement du sang (1) doté d'un circuit sanguin extracorporel, qui comprend une ligne artérielle, une pompe à sang, une unité de traitement du sang et une ligne veineuse, la ligne artérielle et la ligne veineuse pouvant être reliées à un vaisseau sanguin d'un patient et l'appareil de traitement du sang comportant une unité d'évaluation et de commande,

   l'unité d'évaluation et de commande étant conçue pour exécuter les étapes suivantes :

   (a1) détermination de la recirculation du sang dans un vaisseau sanguin du patient relié au circuit sanguin extracorporel ; et
   (b) calcul du flux sanguin dans le vaisseau sanguin en utilisant la recirculation du sang déterminée selon (a1) et une valeur mise à disposition ou également déterminée auparavant pour le débit cardiaque du patient

   et **caractérisé en ce que**

   l'unité d'évaluation et de commande est conçue pour, lors de la détermination du flux sanguin à l'étape (b), prendre en compte, outre la recirculation du sang et le débit cardiaque, le flux sanguin extracorporel et l'écoulement de liquide dans le dispositif de traitement du sang.

2. Appareil de traitement du sang selon la revendication 1, **caractérisé en ce que** l'appareil de traitement du sang comporte en outre un capteur de pression artérielle et **en ce que** l'unité d'évaluation et de commande est conçue pour exécuter en outre l'étape suivante :
   (a2) détermination du débit cardiaque du patient par l'évaluation de la variation temporelle d'un pouls mesuré à l'aide du capteur de pression artérielle.

3. Appareil de traitement du sang selon l'une des revendications précédentes, **caractérisé en ce que** l'appareil de traitement du sang comporte en outre un capteur sous forme de bolus disposé dans la ligne artérielle du circuit sanguin extracorporel et **en ce que** l'unité d'évaluation et de commande est conçue pour exécuter l'étape (a1) de la manière suivante :
   (a1) détermination de la recirculation du sang dans un vaisseau sanguin du patient relié au circuit sanguin extracorporel en utilisant le signal du capteur sous forme de bolus.

4. Appareil de traitement du sang selon l'une des revendications précédentes, **caractérisé en ce que** l'unité d'évaluation et de commande est conçue pour, en supposant que le flux sanguin dans le vaisseau concerné puisse atteindre au maximum une part définie du débit cardiaque, définir des valeurs critiques pour la recirculation pour un raccordement normal et/ou inversé de la ligne artérielle et la ligne veineuse au vaisseau sanguin.

5. Appareil de traitement du sang selon la revendication 4, **caractérisé en ce que** l'unité d'évaluation et de commande est conçue pour comparer la recirculation déterminée avec ces valeurs critiques et la classer en groupes sur la base de la comparaison.

6. Appareil de traitement du sang selon la revendication 5, **caractérisé en ce que** l'appareil comporte en outre une unité d'émission, qui est en liaison avec l'unité d'évaluation et de commande, l'unité d'émission et l'unité d'évaluation et de commande étant conçues pour, selon le groupe auquel appartient la recirculation déterminée, émettre un signal différent pour l'utilisateur.

7. Appareil de traitement du sang selon la revendication 5 ou 6, **caractérisé en ce que** trois groupes sont différentiés :

   (1) la recirculation déterminée est inférieure à la valeur critique pour raccordement normal ;
   (2) la recirculation déterminée est supérieure à la valeur critique pour raccordement normal mais inférieure à la valeur critique pour raccordement inversé ; et
   (3) la recirculation déterminée est supérieure à la valeur critique pour raccordement inversé.

8. Appareil de traitement du sang selon la revendication 7, **caractérisé en ce que** l'unité d'évaluation et de commande est conçue de telle manière que, quand la recirculation déterminée a été associée au groupe (2), la capacité de pompage de la pompe à sang est diminuée et l'exécution des étapes (a1) et (b) est répétée.

9. Appareil de traitement du sang selon l'une des revendications précédentes, **caractérisé en ce que** l'unité d'évaluation et de commande est conçue pour émettre un signal d'avertissement quand le flux sanguin déterminé dans

**EP 3 416 701 B1**

le vaisseau concerné dépasse une valeur seuil supérieure ou est inférieur à une valeur seuil inférieure.

Figur 1

Figur 2

Figur 3

Figur 4

Figur 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011131358 A2
- EP 0773035 B2
- EP 1576341 B1
- DE 102012007081 A1
- US 20110034813 A1
- EP 0569506 B1

**In der Beschreibung aufgeführte Nicht-Patentliteratur**

- **SCHNEDITZ et al.** Measurement of access flow during hemodialysis using the constant infusion approach. *ASAIO journal,* 1998, vol. 44, 74
- **SCHNEDITZ.** Surveillance of Access Function by the Blood Temperature Monitor. *Seminars in Dialysis,* 2003, vol. 16, 483